# EUROPEAN PATENT APPLICATION

(11) **EP 4 843 487 A1**
(43) Date of publication of application: **25.11.2026**
(21) Application number: 25178082.1
(22) Date of filing: 21.05.2025
(51) Int. Cl.: A61B 5/01, G01K 13/20, A61F 7/00, A61M 1/36

(54) **TEMPERATURE SENSING DEVICE**

(71) Applicant: Elmedix NV, 3001 Leuven (BE)
(72) Inventor: BOGERS, Johannes, 2530 Boechout (BE); VAN DEN BOSSCHE, Johan, 3210 Linden (BE); BRANCATO, Luigi, 3010 Leuven (Kessel-Lo) (BE)
(74) Representative: Oryon NV

(57) **Abstract**

A temperature sensing device (1) comprises: an ear plug (2), suitable for insertion in an ear canal (3), comprising at least one circumferential protrusion (21) configured to sealingly contact a wall (31) of the ear canal (3), at least one resistance temperature detector (4) connected to the ear plug (2), configured to measure a temperature inside the ear canal (3), an insulating portion (5) connected to the ear plug (2), configured to be positioned at an opening (30) of the ear canal (3), to thermally insulate the ear canal (3) from the external environment, an external temperature sensor (6), connected to the insulating portion (5), configured to measure an environmental temperature, an output interface (7), connected to the resistance temperature detector (4) and the external temperature sensor (6), configured to transmit the measured temperature data.

## Description

### Field of the Invention

The present invention pertains to a temperature sensing device configured to measure the temperature inside the ear canal.

### Background

International patent application no. WO2006091106A1 relates to a clinical ear thermometer for measuring temperature in a person's ear. A clinical thermometer comprises an ear plug made of a compliant and skin-friendly material and which is shape-adapted to the concha and auditory canal of the person. The ear plug has a thermal radiation detection equipment embedded in the auditory canal part, and the detection equipment is connected to a measurement circuit. The thermometer comprises an infrared radiation pickup element for directing infrared radiation to a receiver element, and the pickup element is arranged at an inner end of the auditory canal part so as to face the person's ear drum, and in that the auditory canal part has a shape and a length to ensure that its inner end is located, in use, at a position between the ear drum and the inner bend of the auditory canal.

The clinical ear thermometer disclosed in WO2006091106A1 is not suitable for providing accurate temperature measurements inside the ear canal.

Us patent application no. US20050209516A1 discloses a probe to measure vital signs. A core body temperature is measured by two sensors, where the first contact sensor positioned on a resilient ear plug and the second sensor is on the external portion of the probe. The second temperature sensor provides a reference signal to a heater that is warmed up close to the body core temperature. The heater is connected to a common heat equalizer for the temperature sensor and the pulse oximeter. Temperature of the heat equalizer enhances the tissue perfusion to improve the optical sensors response. A pilot light is conducted to the ear canal via a contact illuminator, while a light transparent ear plug conducts the reflected lights back to the light detector.

However, the probe disclosed in US20050209516A1 is focused on providing continuous monitoring of various vital signs from the photo-plethysmographic signal. The temperature sensors disclosed in the application are used as a reference signal for a heater and a thermocouple. It would not be possible to remove or modify the optical part, as it is the main focus of the invention of the application.

The present invention aims at resolving the above mentioned problems and providing a more accurate and sensitive temperature sensing device.

### Summary of the Invention

According to the present invention, there is provided a temperature sensing device. The temperature sensing device comprises: an ear plug, suitable for insertion in an ear canal, comprising at least one circumferential protrusion configured to sealingly contact a wall of the ear canal, at least one resistance temperature detector (RTD) connected to the ear plug, configured to measure a temperature inside the ear canal, an insulating portion connected to the ear plug, configured to be positioned at an opening of the ear canal, to thermally insulate the ear canal from an external environment, an external temperature sensor, connected to the insulating portion, configured to measure an environmental temperature, an output interface, connected to the resistance temperature detector and the external temperature sensor, configured to transmit the measured temperature data.

The present disclosure solves the aforementioned technical problems. In fact, the RTD provides a more accurate measurement of the temperature in the ear canal.

According to an embodiment of the present invention, the at least one resistance temperature detector is distanced from the wall of the ear canal. Advantageously, placing the sensor within the plug and surrounding it with soft polymer, not in contact with the ear canal wall, can help in obtaining a more stable and accurate measurement of the ear's internal temperature. The assembly filters out local gradients due to e.g. superficial blood vessels.

More advantageously, the plug increase the comfort of the user. In fact, the epithelium in the ear canal could be irritated by continuous contact with the harder silicone chip of the resistance temperature detector.

More advantageously, the encapsulation protects the device from the drift induced by the diffusion of body fluids, while ensuring the health of the patient.

Still more advantageously, the resistance temperature detector is more protected from the insulating portion.

According to an embodiment of the present invention, the at least one resistance temperature detector is inserted in the ear plug, preferably along a main axis of the ear plug. Advantageously, maximum thickness of insulation material is achieved.

More advantageously, by being on the main longitudinal axis, thus undergoing the least mechanical deformations, there is a reduced likelihood of mechanical damage to the sensor, as the main longitudinal axis undergoes less deformation than the protrusions around it.

According to an embodiment of the present invention, the at least one resistance temperature detector is a platinum resistor thermometer. Advantageously, the platinum resistor thermometer has a high accuracy, high precision, low drift and low self-heating.

According to an embodiment of the present invention, the ear plug has at least an external layer made of polymeric material. Advantageously, the ear plug is flexible and can better adapt to the ear canal.

According to an embodiment of the present invention, the polymeric material is biocompatible. Advantageously, the material can be kept in contact with the ear of an user for extended periods of time.

According to an embodiment of the present invention, the ear plug is configured to adapt its shape to the ear canal. Advantageously, an improved sealing of the ear canal is achieved.

According to an embodiment of the present invention, the insulating portion is made of foam. Advantageously, it is more comfortable for the user.

According to an embodiment of the present invention, the foam is biocompatible. Advantageously, the material can be kept in contact with the ear of an user for extended periods of time.

A system for estimating brain temperature, comprises a temperature sensing device, a processing device, in communication with the output interface of the thermometer, comprising a memory, configured to receive the measured temperature data transmitted by the output interface and a software to estimate deep brain temperature from the received temperature data. Advantageously, the system provides an accurate brain temperature.

According to an embodiment, the software is configured to receive as input at least one temperature measurement of the ear canal and at least one environmental temperature measurement, obtain at least one environment-specific parameter that is indicative of at least one thermal property of said external environment and/or of at least one property of a thermal exchange between a patient's head and said external environment, and calculate an estimate of a deep brain temperature by numerically solving said bioheat model taking said temperature measurement of the ear canal and said environment-specific parameters into account, wherein said bioheat model comprises a plurality of equations representative of an equivalent thermal circuit for modeling heat flows between the brain and the environment. Advantageously, the brain temperature is estimated from real time temperature measurements, increasing the accuracy of the brain temperature estimate.

### Brief description of the drawings

Various technical effects and advantages of embodiments of the present invention will now be described with reference to the accompanying figures, in which:

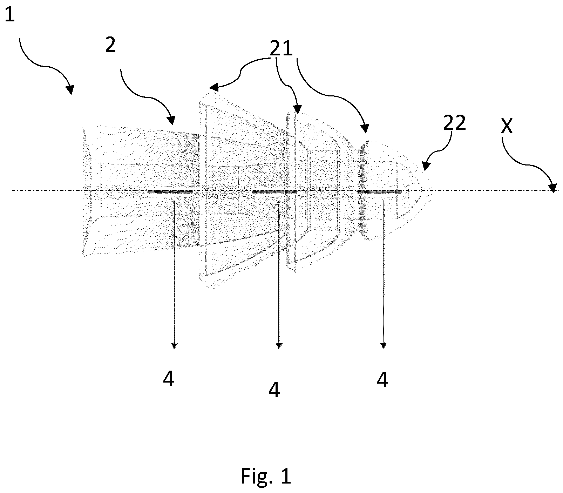

- Figure 1 is a schematic view of the temperature sensor device, according to the present invention, inserted in a user ear canal;
- Figure 2 is a lateral view of a section the temperature sensing device.

### Detailed description of the invention

The present invention will be described with respect to particular embodiments and with reference to certain drawings, but the invention is not limited thereto but only by the claims. Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly, it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

In the description provided herein, numerous specific details are set forth. However, it is understood that embodiments of the invention may be practiced without these specific details. In other instances, well-known methods, structures and techniques have not been shown in detail in order not to obscure an understanding of this description.

The object of the present invention is a temperature sensing device **1.**

The temperature sensing device **1** comprises an ear plug **2,** suitable for insertion in an ear canal **3,** which comprises at least one circumferential protrusion **21** configured to sealingly contact a wall **31** of the ear canal **3.** In other words, the circumferential protrusion **21** is configured to adhere to the wall **31** of the ear canal **3** in order to insulate the ear canal **3.** In a preferred embodiment, the ear plug **2** comprises three circumferential protrusions **21** and at least one is configured to sealingly contact the ear wall **3.**

The temperature sensing device **1** comprises at least one resistance temperature detector **4** connected to the ear plug **2,** configured to measure a temperature inside the ear canal **3.** The at least one resistance temperature detector **4** has higher accuracy and repeatability. Preferably, the temperature sensing device **1** comprises three resistance temperature detector **4.**

The temperature sensing device **1** comprises an insulating portion **5** connected to the ear plug **2,** configured to be positioned at an opening **30** of the ear canal **3,** to thermally insulate the ear canal **3** from the external environment. The insulating portion **5** is configured to reduce temperature fluctuation in the ear canal **3.**

The temperature sensing device **1** comprises an external temperature sensor **6,** connected to the insulating portion **5,** configured to measure an environmental temperature.

The temperature sensing device **1** comprises an output interface **7,** connected to the resistance temperature detector **4** and the external temperature sensor **6,** configured to transmit the measured temperature data. Preferably, the output interface **7** is a lead cable.

Preferably, the at least one resistance temperature detector **4** is distanced from the wall **31** of the ear canal **3.**

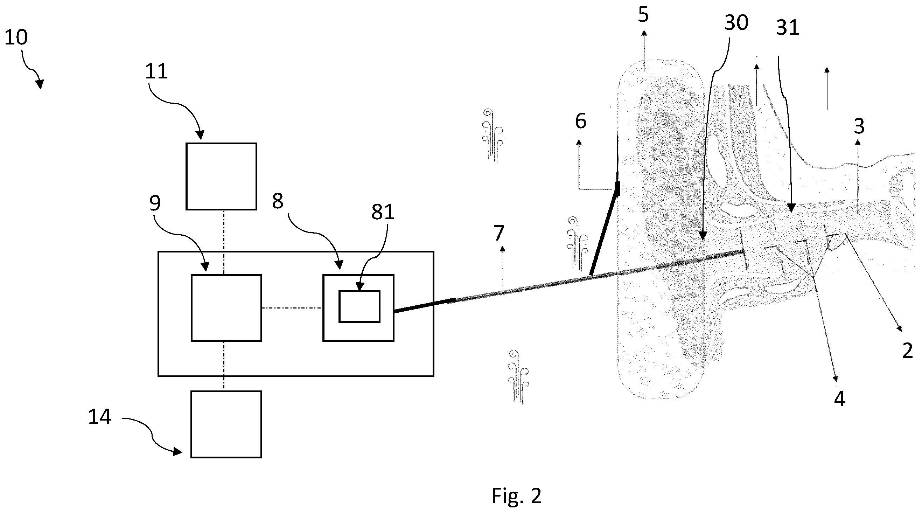

In a preferred embodiment, shown in Figure 2, the at least one resistance temperature detector **4** is inserted in the ear plug **2,** more preferably along a main axis **X** of the ear plug **2.**

Preferably, the RTD sensor is a platinum resistor thermometer. In a preferred embodiment, the platinum resistor thermometer is a

PT1000 or PT100 microsensor. According to a more preferred embodiment, the at least one resistance temperature detector **4** is a PT1000 microsensor. PT1000 sensors are ideal for applications requiring precise temperature measurements.

Preferably, the ear plug **2** has at least an external layer **22** made of polymeric material. Advantageously, the external layer **22** is flexible.

Preferably, the polymeric material is biocompatible. More preferably, the polymeric material can be one of the following: polyurethanes, polyvynilclhorides, polydymethylsiloxane, polyethylenes.

Preferably, the ear plug **2** is configured to adapt its shape to the ear canal **3.** Advantageously, the ear plug **2** can adapt to the ear canal of a patient, and the ear plug **2** provides an optimized minimal stress profile and a thermal sealing of the uniquely shaped ear channel of each patient.

Preferably, the insulating portion **5** is made of foam. More preferably, the foam is biocompatible. According to an embodiment, the biocompatible foam can be one of the following: polyurethanes, polyvynilclhorides, polydymethylsiloxane, polyethylenes.

A system **10** for estimating brain temperature, comprises a temperature sensing device **1,** a processing device **8** and a software **9.** The processing device **8** is in communication with the output interface **7** of the thermometer **1** and comprises a memory **81,** configured to receive the measured temperature data transmitted by the output interface **7.** The software **9** is configured to estimate deep brain temperature from the received temperature data. The estimated deep brain temperature is transmitted to an user via an interface **11.**

The software **9** is configured to receive as input at least one temperature measurement of the ear canal **3** and at least one environmental temperature measurement, obtain at least one environment-specific parameter that is indicative of at least one thermal property of said external environment and/or of at least one property of a thermal exchange between a patient's head and said external environment, calculate an estimate of a deep brain temperature by numerically solving a bioheat model taking said temperature measurement of the ear canal and said environment-specific parameters into account, wherein said bioheat model comprises a plurality of equations representative of an equivalent thermal circuit for modelling heat flows between the brain and the environment.

A use of the system **10** comprises a step of receiving as input at least one temperature measurement of the ear canal **3** and at least one environmental temperature measurement.

The use of the system **10** comprises a step of obtaining at least one environment-specific parameter that is indicative of at least one thermal property of said external environment and/or of at least one property of a thermal exchange between said patient's head and said external environment.

It is already known in the state of the art how to predict a temperature in a human body when that body is in a controllable environment, as disclosed in the application US20220254504A1 of the Applicant.

According to the present invention, the prediction of a temperature is performed on a head of a human person.

The at least one environment-specific parameter may comprise a value representative of a convection coefficient of an airflow to which the patient's head is exposed in the environment. This value may be the convection coefficient indicating the efficiency of convective cooling (or heating) of the patient's head, or may determine the convection coefficient by a known functional relationship or a correspondence look-up table. For example, the value determining the convection coefficient may be representative of a fan speed or fan setting. However, the convection coefficient does not necessarily relate to a forced air flow (e.g. where an average air flow velocity differs substantially from zero), e.g. may also relate to a convection coefficient indicative of air convection in a static air environment (e.g. where an average air velocity is substantially zero).

In one possible use of the system **10**, the parameter could be air velocity distribution in the surrounding of the head of a patient, eventually combined with air temperature and relative humidity.

The at least one environment-specific parameter may comprise an air temperature or a value representative of an air temperature Tₐᵢᵣ of the environment. This air temperature may represent an ambient air temperature of the environment or may represent an air temperature of the air in a forced air flow, e.g. in which heated or cooled air is blown by a fan onto the patient's head.

The use of the system **10** comprises a step of calculating an estimate of a deep brain temperature by numerically solving said bioheat model taking said temperature measurement of the ear canal and said environment-specific parameters into account.

According to an embodiment, the brain temperature is estimated in real time.

According to an alternative embodiment, the estimate of the deep brain temperature is calculated after the temperature measurements of the ear canal and the environment-specific parameters are collected and memorised in the memory **81.**

The environment-specific parameter may be received as input, or may be postulated by the device, e.g. as a trial value in implementing an optimization of the at least one environment-specific parameter to achieve one or more objectives, e.g. the predetermined target temperature and/or a constraint(s) of internal organ temperature(s).

Preferably, the environment-specific parameter is at least one of the following: temperature, humidity and/or convective air flow. Preferably, the environment-specific parameter is measured with at least one of the following: a thermometer, a hygrometer, and/or a hot wire anemometer.

The bioheat model comprises a plurality of equations representative of an equivalent thermal circuit for modelling heat flows between a patient's brain and the environment.

The bioheat model comprises a plurality of equations representative of an equivalent thermal circuit for modelling heat flows in the patient's head and between the body and the controllable environment, in which the equivalent thermal circuit comprises a blood compartment and a plurality of anatomical segments, such as the brain and the ears.

According to an embodiment, a temperature control unit, as disclosed in the Applicant's patent US-12076270-B2 is configured to receive the estimated deep brain temperature, compare the estimated deep brain temperature with a reference brain temperature, generate an alert signal if the estimated deep brain temperature is higher than the reference brain temperature, generate an activation signal configured to activate a refrigeration system, as disclosed in the Applicant's patent US-12076270-B2, to cool down a user. According to different embodiments, cooling could be done via an automatically or manually, for example, by a medically trained person.

The temperature sensing device may also comprise an output for outputting the calculated temperatures, and/or for outputting an optimized value (or values) of the at least one environment-specific parameter, and/or for outputting a control signal for controlling the controllable environment.

Advantageously, in use, when the refrigeration system is activated, it is possible to reduce the temperature of the brain of a user, which means that risks of brain damages are reduced. More advantageously, the refrigeration system improves the safety of the patient by preserving the normal functioning of brain tissue. In fact, the refrigeration system prevents reversible and non-reversible malfunctioning of the brain and associated structure.

## Claims

1. A temperature sensing device (1), comprising:
- an ear plug (2), suitable for insertion in an ear canal (3), comprising at least one circumferential protrusion (21) configured to sealingly contact a wall (31) of the ear canal (3),
- at least one resistance temperature detector (4) connected to the ear plug (2), configured to measure a temperature inside the ear canal (3),
- an insulating portion (5) connected to the ear plug (2), configured to be positioned at an opening (30) of the ear canal (3), to thermally insulate the ear canal (3) from the external environment,
- an external temperature sensor (6), connected to the insulating portion (5), configured to measure an environmental temperature,
- an output interface (7), connected to the resistance temperature detector (4) and the external temperature sensor (6), configured to transmit the measured temperature data.

2. A temperature sensing device (1), according to claim 1, wherein the at least one resistance temperature detector (4) is distanced from the wall (31) of the ear canal (3).

3. A temperature sensing device (1), according to claims 1 or 2, wherein the at least one resistance temperature detector (4) is inserted in the ear plug (2), preferably along a main axis of the ear plug (2).

4. A temperature sensing device (1), according to any claims 1-3, wherein the at least one resistance temperature detector (4) is a platinum resistor thermometer.

5. A temperature sensing device (1), according to any claims 1-4, wherein the ear plug (2) has at least an external layer (22) made of polymeric material.

6. A temperature sensing device (1), according to claim 5, wherein the polymeric material is biocompatible.

7. A temperature sensing device (1), according to any claims 1-6, wherein the ear plug (2) is configured to adapt its shape to the ear canal (3).

8. A temperature sensing device (1), according to any claims 1-7, wherein the insulating portion is made of foam.

9. A temperature sensing device (1), according to claim 8, wherein the foam is biocompatible.

10. A system (10) for estimating brain temperature, comprising:
- a temperature sensing device (1), according to claims 1-9,
- a processing device (8), in communication with the output interface (7), comprising a memory (81), configured to receive the measured temperature data transmitted by the output interface (7) and a software (9) to estimate deep brain temperature from the received temperature data.

11. A system (10), according to claim 10, wherein the software (9) is configured to:
- receive as input at least one temperature measurement of the ear canal (30 and at least one environmental temperature measurement,
- obtain at least one environment-specific parameter that is indicative of at least one thermal property of said external environment and/or of at least one property of a thermal exchange between a body of a patient and said external environment, and
- calculate an estimate of a deep brain temperature by numerically solving a bioheat model taking said temperature measurement of the ear canal and said environment-specific parameters into account, wherein said bioheat model comprises a plurality of equations representative of an equivalent thermal circuit for modeling heat flows between the brain and the environment.

12. Use of the system (10) of claims 10-11, comprising the steps of:
- receiving as input at least one temperature measurement of the ear canal and at least one environmental temperature measurement,
- obtaining at least one environment-specific parameter that is indicative of at least one thermal property of said external environment and/or of at least one property of a thermal exchange between said body and said external environment, and
- calculating an estimate of a deep brain temperature by numerically solving said bioheat model taking said temperature measurement of the ear canal and said environment-specific parameters into account,
wherein said bioheat model comprises a plurality of equations representative of an equivalent thermal circuit for modeling heat flows between the brain and the environment.

13. A temperature control unit (12) configured to:
- receive the estimated deep brain temperature, from the system (10) of claims 10-11,
- compare the estimated deep brain temperature with a reference brain temperature,
- generate an alert signal if the estimated deep brain temperature is higher than the reference brain temperature,
- generate an activation signal configured to activate a refrigeration system to cool down a user.
